(19) Europäisches Patentamt
European Patent Office
Office européen des brevets

(11) Veröffentlichungsnummer: **0 021 114**
**B1**

(12) **EUROPÄISCHE PATENTSCHRIFT**

(45) Veröffentlichungstag der Patentschrift: **18.05.83**

(51) Int. Cl.³: **C 07 C 121/46,** C 07 C 121/407

(21) Anmeldenummer: **80103022.2**

(22) Anmeldetag: **30.05.80**

(54) **Verfahren zur Herstellung von 2-Cyano-3,3-dimethyl-cyclopropan-1-carbonsäureestern, Zwischenprodukte hierfür und deren Herstellung.**

(30) Priorität: **12.06.79 DE 2923777**

(43) Veröffentlichungstag der Anmeldung: **07.01.81 Patentblatt 81/1**

(45) Bekanntmachung des Hinweises auf die Patenterteilung: **18.05.83 Patentblatt 83/20**

(84) Benannte Vertragsstaaten: **BE CH DE FR GB IT LI NL**

(56) Entgegenhaltungen:
**AT-B-291 215**
**DE-A-2 621 831**
**DE-A-2 621 835**
**DE-A-2 724 734**
**DE-A-2 732 213**
**DE-A-2 926 852**
**FR-A-2 313 347**
**GB-A-1 553 818**
**US-A-3 843 709**

(73) Patentinhaber: **BAYER AG, Zentralbereich Patente, Marken und Lizenzen, D-5090 Leverkusen 1, Bayerwerk (DE)**

(72) Erfinder: **Maurer, Fritz, Dr., Roeberstrasse 8, D-5600 Wuppertal 1 (DE)**
Erfinder: **Riebel, Hans-Jochem, Dr., In der Beek 92, D-5600 Wuppertal 1 (DE)**
Erfinder: **Priesnitz, Uwe, Dr., Heinrich-Heine-Strasse 42, D-4750 Unna-Massen (DE)**

**EP 0 021 114 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 2-Cyano-3,3-dimethylcyclopropan-1-carbonsäureestern, Zwischenprodukte hierfür und deren Herstellung

Die Erfindung betrifft ein neues Verfahren zur Herstellung von teilweise bekannten 2-Cyano-3,3-dimethylcyclopropan-1-carbonsäureestern, sowie 3-Halogen-3-cyano-2,2-dimethyl-propan-1-carbonsäureester als neue Zwischenprodukte in diesem Verfahren und ein Verfahren zu deren Herstellung.

Es ist bereits bekannt, dass man 3,3-Dimethylcyclopropan-1,2-dicarbonsäure(Caronsäure) erhält, wenn man 2,2-Dimethylpropan-1,3-dicarbonsäureanhydrid(β,β-Dimethylglutarsäureanhydrid) nacheinander mit Phosphorpentabromid, Brom und Alkohol umsetzt, den hierbei gebildeten 1-Brom-2,2-dimethylpropan-1,3-dicarbonsäureester mit Kaliumcarbonat in Alkohol behandelt, nach Abdestillieren des Alkohols den Rückstand in Wasser löst und nach Ansäuern das Produkt mit Äther extrahiert [vgl. „J. Chem. Soc." (London) *75* (1899), 49-61].

Diese Caronsäuresynthese ist mit dem Nachteil behaftet, dass man in der Bromierungsstufe neben 1-Brom-2,2-dimethylpropan-1,3-dicarbonsäureester als Nebenprodukt etwa 20-30% 1,3-Dibrom-2,2-dimethyl-1,3-propandicarbonsäureester erhält, welcher nicht sauber abgetrennt werden kann. Die Ausbeute des gewünschten Produktes wird hierdurch stark vermindert. Ein weiterer Nachteil der Synthesemethode liegt in der Verwendung der teuren Reagentien Phosphorpentabromid und Brom.

Weiter ist bekannt, dass man 2-Cyano-3,3-dimethylcyclopropan-1-carbonsäureester, welcher leicht zu Caronsäure verseift werden kann, erhält, wenn man 2-Cyano-3,3-dimethylcyclopropan-1,1-dicarbonsäureester decarboxyliert [vgl. JP-PS Nr. 53.108951 und „Bull. Soc. Chim. Belg.", *87* (1978) 721-732]. Die Ausbeuten bei diesem Verfahren sind jedoch ebenfalls unbefriedigend.

Aus GB-P Nr. 1553818 war bekannt 2-(2,2-Dichlorvinyl)-2,2-dimethylcyclopropancarbonsäurenitril durch Cyclisierung und Dehydrohalogenierung aus 4-Brom-3,3-dimethyl-6-trichlorhexancarbonsäurenitrol herzustellen. Der Nachteil dieser Reaktion liegt darin, dass sie nur zu dichlorvinylsubstituierten Cyclopropanverbindungen führt. Änderungen an der Seitenkette sind nur durch Abbau derselben möglich. Ein weiterer Nachteil des Verfahrens liegt in den vielen Stufen, die zur Herstellung der Ausgangsverbindungen durchgeführt werden müssen. Dasselbe trifft zu für den aus der FR-P Nr. 2313347, DOS Nrn. 2621831, 2621835 und 2732213 bekannten Stand der Technik.

Aus US-P Nr. 3843709 war die Cyclisierung von 4-Chlorbutancarbonsäurenitril zum entsprechenden Cyclopropancyanid bekannt. Es geht daraus jedoch nichts über die Anwendbarkeit der Reaktion zur Herstellung von substituierten Cyclopropancarbonsäuren hervor.

Die vorliegende Erfindung betrifft

1) ein Verfahren zur Herstellung von 2-Cyano-3,3-dimethylcyclopropan-1-carbonsäureestern der Formel I:

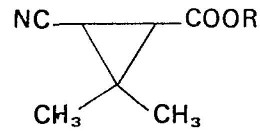

(I)

in welcher R für gegebenenfalls substituiertes Alkyl steht, dadurch gekennzeichnet, dass man 3-Carboxy-3-cyano-2,2-dimethylpropan-1-carbonsäureester der Formel III:

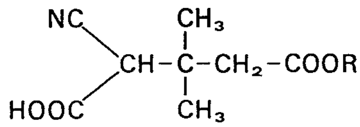

(III)

in welcher R die oben angegebene Bedeutung hat, mit einem Halogenierungsmittel, gegebenenfalls in Gegenwart eines Säureakzeptors und gegebenenfalls unter Verwendung eines Verdünnungsmittels, bei Temperaturen zwischen −20 und +100°C umsetzt und das intermediär gebildete Halogenierungsprodukt der Formel IV:

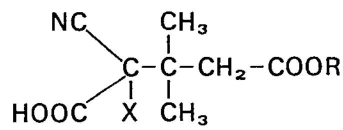

(IV)

in welcher R die oben genannte Bedeutung hat, und X für Halogen steht, durch Erhitzen auf Temperaturen zwischen 150 und 250°C decarboxyliert und anschliessend den so erhaltenen 3-Halogen-3-cyano-2,2-dimethylpropan-1-carbonsäureester der Formel II:

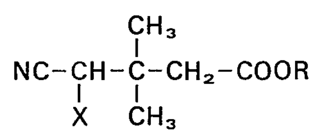

(II)

in welcher R die oben angegebene Bedeutung hat, und X für Halogen steht, mit einer Base, gegebenenfalls in Gegenwart eines Verdünnungsmittels, bei Temperaturen zwischen 0 und 100°C umsetzt;

2) neue 3-Halogen-3-cyano-2,2-dimethylpropan-1-carbonsäureester der Formel II:

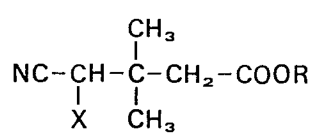

(II)

in welcher R für gegenbenenfalls substituiertes Alkyl steht, und X für Halogen steht;

3) ein Verfahren zur Herstellung von 3-Halogen-3-cyano-2,2-dimethylpropan-1-carbonsäureestern der Formel II (oben), dadurch gekennzeichnet, dass man 3-Carboxy-3-cyano-2,2-dimethylpropan-1-carbonsäureester der Formel II:

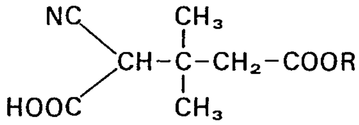
(III)

in welcher R die oben angegebene Bedeutung hat, mit einem Halogenierungsmittel, gegebenenfalls in Gegenwart eines Säureakzeptors und gegebenenfalls unter Verwendung eines Verdünnungsmittels, bei Temperaturen zwischen −20 und +100°C umsetzt und das intermediär gebildete Halogenierungsprodukt der Formel IV:

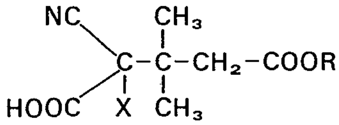
(IV)

in welcher R und X die oben angegebenen Bedeutungen haben, durch Erhitzen auf Temperaturen zwischen 150 und 250°C decarboxyliert.

Überraschenderweise können nach dem erfindungsgemässen Verfahren 1, 2-Cyano-3,3-dimethylcyclopropan-1-carbonsäureester der Formel I wesentlich einfacher und kostengünstiger als nach bekannten Methoden in guten Ausbeuten und in hoher Reinheit hergestellt werden. Caronsäure, welche als Zwischenprodukt für Pyrethroide verwendet werden kann, erhält man daraus nahezu quantitativ in einer einfachen Verseifungsreaktion.

Verwendet man bei Verfahren 1 beispielsweise 3-Chlor-3-cyano-2,2-dimethylpropan-1-carbonsäuremethylester als Ausgangsstoff und Natriummethylat als Base, so kann der Reaktionsablauf durch folgendes Formelschema skizziert werden.

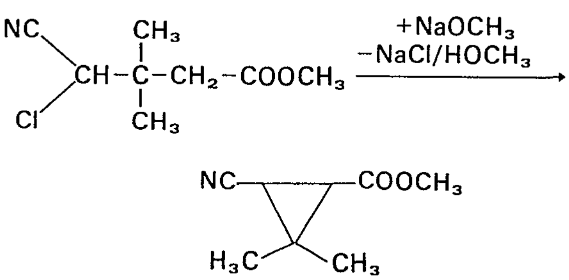


Die bei Verfahren 1 als Ausgangsverbindungen zu verwendenden neuen 3-Halogen-3-cyano-2,2-dimethylpropan-1-carbonsäureester sind durch Formel II definiert. Vorzugsweise stehen darin R für $C_1$ bis $C_4$-Alkyl, insbesondere für Methyl oder Äthyl und Isopropyl, und X für Chlor oder Brom.

Als Beispiele seien genannt:

3-Chlor-3-cyano-2,2-dimethylpropan-1-carbonsäuremethylester, -äthylester, -n-propylester, -isopropylester, -n-butylester, -isobutylester, und -tert.-butylester sowie

3-Brom-3-cyano-2,2-dimethylpropan-1-carbonsäuremethylester, -äthylester, -n-propylester, isopropylester, -n-butylester, -isobutylester, -sek.-butylester und -tert.-butylester.

Das erfindungsgemässe Verfahren 1 wird vorzugsweise unter Verwendung von Verdünnungsmitteln durchgeführt. Als solche kommen insbesondere Wasser und/oder polare organische Lösungsmittel in Betracht. Hierzu gehören insbesondere Carbonsäureamide, wie z.B. Dimethylformamid und N-Methylpyrrolidon, Sulfoxide und Sulfone, wie z.B. Dimethylsulfoxid und Tetramethylensulfon, Phosphorsäureamide, wie z.B. Hesamethylphosphorsäuretriamid, Äther, wie z.B. Glycoldimethyläther, Diglycoldimethyläther, Tetrahydrofuran und Dioxan, Nitrile, wie z.B. Acetonitril und Propionitril, sowie Alkohole, wie z.B. Methanol, Äthanol, n- und -iso-Propanol, n-, iso-, sek.- und tert.-Butanol.

Letztere werden als Verdünnungsmittel für Verfahren 1 besonders bevorzugt.

Die Reaktionstemperatur wird bei Verfahren 1 zwischen 0 und 100°C, vorzugsweise zwischen 10 und 80°C, gehalten. Die Umsetzung wird bei Normaldruck oder einem dem Dampfdruck des Verdünnungsmittels angepassten Druck durchgeführt.

Als Basen können bei Verfahren 1 die üblichen Säurebindemittel verwendet werden. Besonders geeignet sind Alkalicarbonate und -alkoholate, wie Natrium- und Kaliummethylat bzw. -äthylat, ferner aliphatische, aromatische oder heterocyclische Amine, beispielsweise Triäthylamin, Trimethylamin, Dimethylanilin, Dimethylbenzylamin, Pyridin und Diazabicyclononan.

Auf 1 mol 3-Halogen-3-cyano-2,2-dimethylpropan-1-carbonsäureester der Formel II setzt man im allgemeinen zwischen 1,0 und 1,5 mol, vorzugsweise zwischen 1,05 und 1,3 mol Base, ein.

In einer bevorzugten Ausführungsform des erfindungsgemässen Verfahrens wird der 3-Halogen-3-cyano-2,2-dimethylpropan-1-carbonsäureester II in einem der oben angegebenen Verdünnungsmittel gelöst und diese Lösung wird mit der Base, welche gegebenenfalls in einem der oben angegebenen Verdünnungsmittel gelöst ist, tropfenweise versetzt. Das komplette Reaktionsgemisch wird einige Stunden bei einer Temperatur zwischen 40 und 70°C gerührt.

Die Aufarbeitung erfolgt auf übliche Weise: Man versetzt das Reaktionsgemisch mit Wasser und extrahiert mit einem mit Wasser nicht mischbaren organischen Lösungsmittel, wie z.B. Methylenchlorid. Die organische Phase wird mit verdünnter Salzsäure und mit Wasser gewaschen, getrocknet, filtriert und eingeengt. Das zurückbleibende Rohprodukt kann durch Vakuumdestillation gereinigt werden. Zur Charakterisierung dient der Siedepunkt.

Die nach dem erfindungsgemässen Verfahren 1 herzustellenden 2-Cyano-3,3-dimethylcyclopropan-1-carbonsäureester können durch Hydrolyse, beispielsweise durch Erhitzen mit Alkalilaugen, wie z.B. 15% Natronlauge, auf Temperaturen zwischen 80 und 120°C, und anschliessendes Ansäuern bei Raumtemperatur mit starken Säuren, wie z.B. Salzsäure, in 3,3-Dimethylcyclopropan-1,2-dicarbonsäure (Caronsäure) umgewandelt

werden. Caronsäure fällt hierbei kristallin an und kann durch Filtration isoliert werden.

Caronsäure oder deren Ester können als Zwischenprodukte zur Herstellung von insektizid und akarizid wirksamen Pyrethroiden verwendet werden [vgl. „Pestic. Sci.", *7* (1976), 492-498; „Tetrahedron Lett.", *1978*, 1847-1850].

Verwendet man bei Verfahren 3 als Ausgangsverbindung beispielsweise 3-Carboxy-3-cyano-2,2-dimethylpropan-1-carbonsäuremethylester sowie als Halogenierungsmittel Chlor und als Säureakzeptor Natriumacetat, so kann der Reaktionsablauf durch folgendes Formelschema skizziert werden:

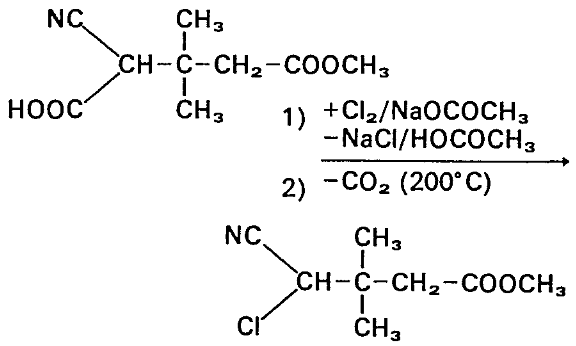


Man erhält die neuen 3-Halogen-3-cyano-2,2-dimethylpropan-1-carbonsäureester nach dem oben unter 3 beschriebenen Verfahren durch Umsetzung von 3-Carboxy-3-cyano-2,2-dimethylpropan-1-carbonsäureestern der Formel III (oben) mit einem Halogenierungsmittel, gegebenenfalls in Gegenwart eines Säureakzeptors und gegebenenfalls unter Verwendung eines Verdünnungsmittels, vorzugsweise von Wasser, bei Temperaturen zwischen −20 und +100°C, vorzugsweise zwischen 0 und 50°C, und anschliessende Decarboxylierung des intermediär gebildeten Halogenierungsproduktes der Formel IV (oben) durch Erhitzen auf Temperaturen zwischen 150 und 250°C, vorzugsweise zwischen 180 und 220°C.

Als Halogenierungsmittel, welche bei Verfahren 3 zu verwenden sind, seien beispielsweise Brom, Chlor und Sulfurylchlorid genannt. Vorzugsweise werden Brom und Chlor verwendet.

Als Säureakzeptoren, welche bei Verfahren 3 verwendet werden können, seien Alkali- und Erdalkalihydroxide, wie z.B. Natrium-, Kalium- und Calciumhydroxid, Alkali- und Erdalkalioxide und -carbonate bzw. -hydrogencarbonat, wie Calciumoxid und -carbonat, ferner Alkaliacetate, wie z.B. Natrium- und Kaliumacetat genannt. Letztere werden vorzugsweise verwendet.

In einer bevorzugten Ausführungsform von Verfahren 3 wird der als Ausgangsverbindung einzusetzende 3-Carboxy-3-cyano-2,2-dimethylpropan-1-carbonsäureester der Formel III mit 2 bis 3 Moläquivalenten eines der oben genannten Säureakzeptoren in Wasser vorgelegt und unter Rühren bei einer Temperatur zwischen 0 und 50°C 1,0 bis 1,5, vorzugsweise 1,05 bis 1,3 Moläquivalente, Halogenierungsmittel (Brom oder Chlor) eindosiert. Nach mehrstündigem Rühren des kompletten Reaktionsgemisches wird mit einer starken Säure, wie z.B. Salzsäure, angesäuert und mit einem mit Wasser nicht mischbaren Lösungsmittel, wie z.B. Methylenchlorid, extrahiert. Die organische Phase wird nach Trocknen und Filtration eingeengt und der Rückstand tropfenweise in einen auf 180 bis 220°C erhitzten Kolben gegeben. Das hierbei gebildete Decarboxylierungsprodukt der Formel II kann durch Vakuumdestillation in reiner Form isoliert werden.

Die bei Verfahren 3 als Ausgangsstoffe einzusetzenden 3-Carboxy-3-cyano-2,2-dimethylpropan-1-carbonsäureester sind durch Formel III definiert.

Vorzugsweise steht darin R für $C_1$ bis $C_4$-Alkyl, insbesondere für Methyl, Äthyl und Isopropyl.

Als Beispiele seien genannt:

3-Carboxy-3-cyano-2,2-dimethylpropan-1-carbonsäuremethylester, -äthylester, -n-propylester, -isopropylester, -n-butylester, -isobutylester, -sek.-butylester und -tert.-butylester.

3-Carboxy-3-cyano-2,2-dimethylpropan-1-carbonsäureester der Formel III sind bekannt [vgl. „J. Chem. Soc." (London/*75* (1899), 49-61].

*Beispiel 1*

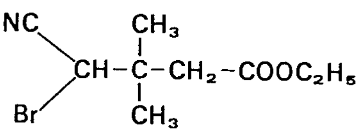

Zu einer Lösung von 42,6 g (0,2 mol) 3-Carboxy-3-cyano-2,2-dimethylpropan-1-carbonsäureäthylester und 41 g Natriumacetat in 400 ml Wasser werden bei 20°C 40 g (0,25 mol) Brom getropft. Es wird 5 h bei 20°C nachrühren gelassen. Dann wird mit verdünnter HCl angesäuert und zweimal mit je 100 ml Methylenchlorid extrahiert.

Diese Lösung wird über Natriumsulfat getrocknet und dann eingeengt. Der Rückstand wird zunächst in einen auf 200°C erhitzten Kolben getropft, wobei eine kontinuierliche $CO_2$-Entwicklung einsetzt und dann zweimal fraktioniert destilliert.

Man erhält 38 g (76,5% der Theorie) 3-Brom-3-cyano-2,2-dimethylpropan-1-carbonsäureäthylester in Form eines farblosen Öls vom Kp. 92-95°C/2,5 mmHg.

*Beispiel 2*

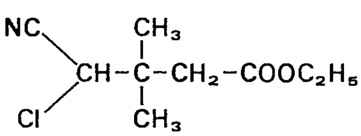

Zu einer Lösung von 42,6 g (0,2 mol) 3-Carboxy-3-cyano-2,2-dimethylpropan-1-carbonsäureäthylester und 41 g Natriumacetat in 400 ml Wasser werden bei 20°C 17,8 g (0,25 mol) Chlor eingeleitet. Es wird 5 h bei 20°C nachgerührt, dann wird mit verdünnter HCl angesäuert.

Die Salzsäure Lösung wird wie unter Beispiel 1 beschrieben aufgearbeitet.

Man erhält 28 g (69% der Theorie) 3-Chlor-3-cyano-2,2-dimethylpropan-1-carbonsäureäthylester in Form eines farblosen Öls vom Kp. 80-82°C/1 mmHg.

*Beispiel 3*

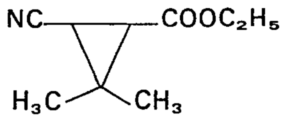

Zu einer Lösung von 24,8 g (0,1 mol) 3-Brom-3-cyano-2,2-dimethylpropan-1-carbonsäureäthylester in 100 ml Alkohol werden bei 20°C 14,8 g (0,12 mol) Diazabicyclononan getropft. Man erwärmt anschliessend 3 h auf 50-60°C kühlt ab auf 20°C und versetzt das Reaktionsgemisch mit 200 ml Wasser.

Es wird zweimal mit je 100 ml Methylenchlorid extrahiert. Die vereinigten Methylenchloridextrakte werden zweimal mit je 50 ml 5%iger Salzsäure und zweimal mit je 50 ml Wasser gewaschen, über Natriumsulfat getrocknet und eingeengt. Der Rückstand wird fraktioniert.

Man erhält 12,5 g (75% der Theorie) 2-Cyano-3,3-dimethylcyclopropan-1-carbonsäureäthylester in Form eines farblosen Öls vom Kp. 62-64°C/1 mmHg.

*Beispiel 4*

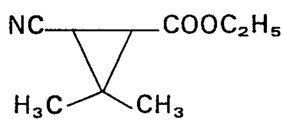

Zu einer Lösung von 24,8 g (0,1 Mol) 3-Brom-3-cyano-2,2-dimethylpropan-1-carbonsäureäthylester in 50 ml Äthanol werden 8,15 g Natriumäthylat (0,12 mol) in 30 ml Äthanol getropft. Es wird 3 h bei 50-60°C nachgerührt. Dann wird das Reaktionsgemisch mit 200 ml Wasser versetzt, mit HCl neutralisiert und zweimal mit je 50 ml Methylenchlorid extrahiert. Die vereinigten Methylenchloridextrakte werden zweimal mit je 50 ml $H_2O$ gewaschen, über $Na_2SO_4$ getrocknet und dann eingeengt. Der Rückstand wird fraktioniert.

Man erhält 11,5 g (69% der Theorie) 2-Cyano-3,3-dimethylcyclopropan-1-carbonsäureäthylester in Form eines farblosen Öls vom Kp. 68-72°C/ 2 mmHg.

*Beispiel 5*

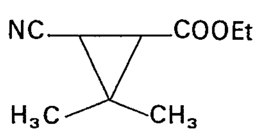

Zu einer Lösung von 20,3 g (0,1 mol) 3-Chlor-3-cyano-2,2-dimethylpropan-1-carbonsäureäthylester in 500 ml Äthanol werden 8,15 g (0,12 mol) Natriumäthylat getropft. Es wird 4 h bei 60°C nachgerührt und dann wie in Beispiel 4 beschrieben aufgearbeitet.

Ausbeute: 14 g (84% des Theorie) 2-Cyano-3,3-dimethylcyclopropan-1-carbonsäureäthylester.

*Beispiel 6*

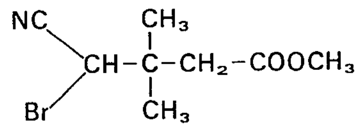

Analog Beispiel 1 kann 3-Brom-3-cyano-2,2-dimethylpropan-1-carbonsäuremethylester in einer Ausbeute von 75% der Theorie hergestellt werden (Kp. 83°C/ 1 mmHg).

*Beispiel 7*

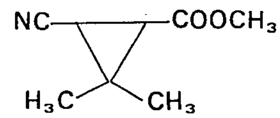

Analog Beispiel 4 kann 2-Cyano-3,3-dimethylcyclopropan-1-carbonsäuremethylester aus dem Bromid in Beispiel 6 und Natriummethylat als Base hergestellt werden in einer Ausbeute von 75% der Theorie (Kp. 62°C/ 1 mmHg).

Beispiel für die Weiterverarbeitung:

*Beispiel 8*

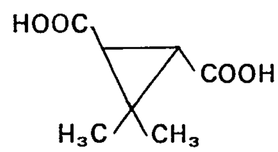

Eine Mischung von 50 ml 15% Natronlauge und 8,5 g (0,05 mol) 3,3-Dimethyl-2-cyanocyclopropan-1-carbonsäureäthylester wird 18 h unter Rückfluss gekocht. Dann kühlt man das Gemisch auf 10°C ab, gibt konz. Salzsäure zu, bis ein pH-Wert von ca. 2 erreicht ist, und kühlt dann 1 h im Eisbad. Das ausgefallene Produkt wird abgesaugt und mit Eiswasser nachgewaschen. Man erhält so 7,3 g (92% der Theorie) trans-3,3-Dimethylcyclopropan-1,2-dicarbonsäure in Form eines farblosen Pulvers mit dem Schmelzpunkt 217°C.

**Patentansprüche**

1. Verfahren zur Herstellung von 2-Cyano-3,3-dimethylcyclopropan-1-carbonsäureestern der Formel I:

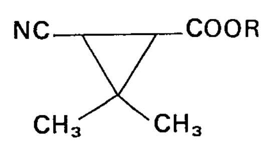

(I)

in welcher:

R für gegebenenfalls substituiertes Alkyl steht, dadurch gekennzeichnet, dass man 3-Carboxy-3-cyano-2,2-dimethylpropan-1-carbonsäureester der Formel III:

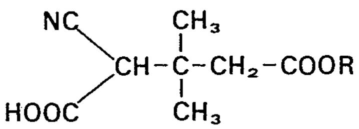

(III)

in welcher:
R die oben angegebene Bedeutung hat,
mit einem Halogenierungsmittel, gegebenenfalls in Gegenwart eines Säureakzeptors und gegebenenfalls unter Verwendung eines Verdünnungsmittels, bei Temperaturen zwischen −20 und +100°C umsetzt und das intermediär gebildete Halogenierungsprodukt der Formel IV:

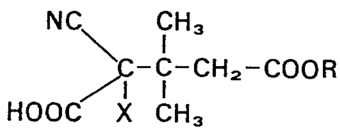

(IV)

in welcher:
R die obengenannte Bedeutung hat, und
X für Halogen steht,
durch Erhitzen auf Temperaturen zwischen 150 und 250°C decarboxyliert und anschliessend den so erhaltenen 3-Halogen-3-cyano-2,2-dimethyl-propan-1-carbonsäureester der Formel II:

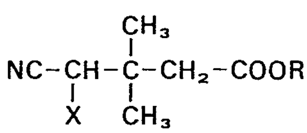

(II)

in welcher:
R die oben angegebene Bedeutung hat, und
X für Halogen steht,
mit einer Base, gegebenenfalls in Gegenwart eines Verdünnungsmittels, bei Temperaturen zwischen 0 und 100°C umsetzt.

2. 3-Halogen-3-cyano-2,2-dimethylpropan-1-carbonsäureester der Formel II:

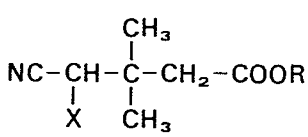

(II)

in welcher:
R für gegebenenfalls substituiertes Alkyl steht, und
X für Halogen steht.

3. Ein Verfahren zur Herstellung von 3-Halogen-3-cyano-2,2-dimethylpropan-1-carbonsäureestern der Formel II:

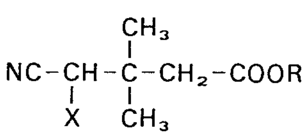

(II)

in welcher:
R für gegebenenfalls substituiertes Alkyl steht, und
X für Halogen steht,
dadurch gekennzeichnet, dass man 3-Carboxy-3-cyano-2,2-dimethylpropan-1-carbonsäureester der Formel III:

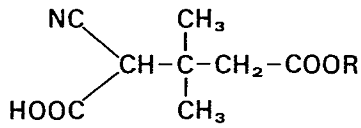

(III)

in welcher:
R die oben angegebene Bedeutung hat,
mit einem Halogenierungsmittel, gegebenenfalls in Gegenwart eines Säureakzeptors und gegebenenfalls unter Verwendung eines Verdünnungsmittels, bei Temperaturen zwischen −20 und +100°C umsetzt, und das intermediär gebildete Halogenierungsprodukt der Formel IV:

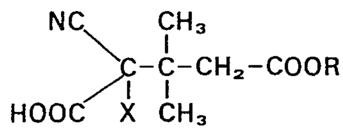

(IV)

in welcher:
R und X die oben angegebenen Bedeutungen haben
durch Erhitzen auf Temperaturen zwischen 150 und 250°C decarboxyliert.

4. Verwendung von 3-Halogen-3-cyano-2,2-dimethylpropan-1-carbonsäureestern der Formel II gemäss Anspruch 2 zur Herstellung von 2-Cyano-3,3-dimethylcyclopropan-1-carbonsäureestern der Formel I:

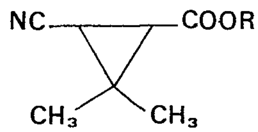

(I)

in welcher:
R für gegebenenfalls substituiertes Alkyl steht,
dadurch gekennzeichnet, dass man 3-Halogen-3-cyano-2,2-dimethylpropan-1-carbonsäureester der Formel II:

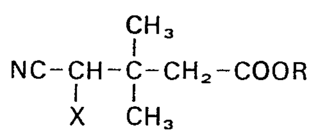

(II)

in welcher:
R die oben angegebene Bedeutung hat, und
X für Halogen steht,
mit einer Base, gegebenenfalls in Gegenwart eines Verdünnungsmittels, bei Temperaturen zwischen 0 und 100°C umsetzt.

## Claims

1. Process for the preparation of 2-cyano-3,3-dimethylcyclopropane-1-carboxylic acid esters of the formula I:

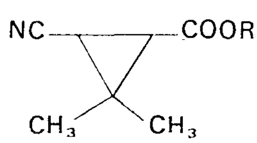

(I)

in which:
R represents optionally substituted alkyl, characterised in that 3-carboxy-3-cyano-2,2-dimethylpropane-1-carboxylic acid esters of the formula III:

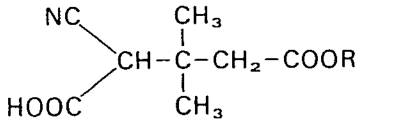
(III)

in which:
R has the meaning indicated above, are reacted with a halogenating agent, if appropriate in the presence of an acid acceptor and if appropriate using a diluent, at temperatures between −20 and +100° C, and the halogenation product, formed as an intermediate, of the formula IV:

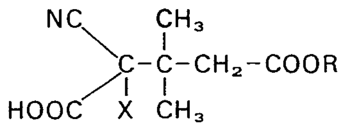
(IV)

in which:
R has the above-mentioned meaning, and
X represents halogen,
is decarboxylated by heating to temperatures between 150 and 250°C and then the 3-halogeno-3-cyano-2,2-dimethylpropane-1-carboxylic acid esters thus obtained, of the formula II:

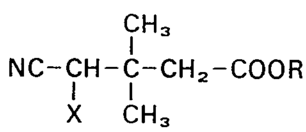
(II)

in which:
R has the meaning indicated above, and
X represents halogen,
are reacted with a base, if appropriate in the presence of a diluent, at temperatures between 0 and 100° C.

2. 3-Halogeno-3-cyano-2,2-dimethylpropane-1-carboxylic acid esters of the formula II:

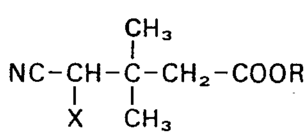
(II)

in which:
R represents optionally substituted alkyl, and
X represents halogen.

3. A process for the preparation of 3-halogeno-3-cyano-2,2-dimethylpropane-1-carboxylic acid esters of the formula II:

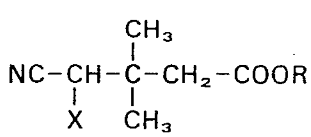
(II)

in which:
R represents optionally substituted alkyl, and
X represents halogen,
characterised in that 3-carboxy-3-cyano-2,2-dimethylpropane-1-carboxylic acid esters of the formula III:

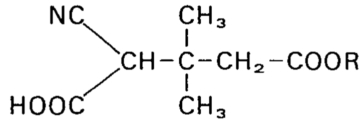
(III)

in which:
R has the meaning indicated above, are reacted with a halogenating agent, if appropriate in the presence of an acid acceptor and if appropriate using a diluent, at temperatures between −20 and +100° C and the halogenation product, formed as an intermediate, of the formula IV:

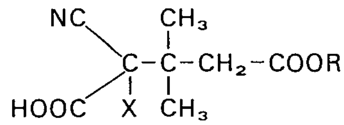
(IV)

in which:
R and X have the meanings indicated above, is decarboxylated by heating to temperatures between 150 and 250° C.

4. Use of 3-halogeno-3-cyano-2,2-dimethylpropane-1-carboxylic acid esters of the formula II according to claim 2 for the preparation of 2-cyano-3,3-dimethylcyclopropane-1-carboxylic acid esters of the formula I:

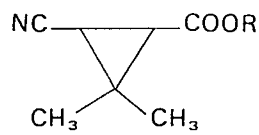
(I)

in which:
R represents optionally substituted alkyl, characterised in that 3-halogeno-3-cyano-2,2-dimethylpropane-1-carboxylic acid esters of the formula II:

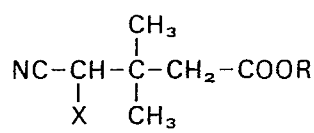
(II)

in which:
R has the meaning indicated above, and
X represents halogen,
are reacted with a base, if appropriate in the presence of a diluent, at temperatures between 0 and 100° C.

## Revendications

1. Procédé de production d'esters de l'acide 2-cyano-3,3-diméthylcyclopropane-1-carboxylique de formule I:

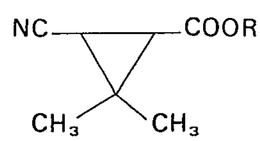
(I)

dans laquelle:
R représente un groupe alkyle éventuellement substitué, caractérisé en ce qu'on fait réagir à des températures comprises entre −20 et +100°C un ester d'acide 3-carboxy-3-cyano-2,2-diméthyl-propane-1-carboxylique de formule III:

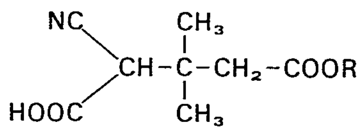
(III)

dans laquelle:
R a le sens précité,
avec un agent d'halogénation, éventuellement en présence d'un accepteur d'acide et éventuellement en utilisant un diluant, et l'on décarboxyle, par chauffage jusqu'à des températures comprises entre 150 et 250°C, le produit d'halogénation, formé de manière intermédiaire et répondant à la formule IV:

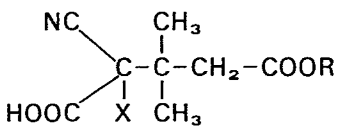
(IV)

dans laquelle:
R a le sens précité, et
X représente un atome d'halogène,
et l'on fait ensuite réagir, à des températures comprises entre 0 et 100°C, l'ester d'acide 3-halogéno-3-cyano-2,2-diméthylpropane-1-carboxylique ainsi obtenu, de formule II:

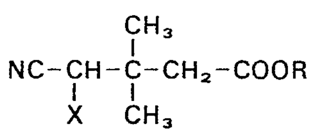
(II)

dans laquelle:
R a le sens précité, et
X représente un atome d'halogène
avec une base, éventuellement en présence d'un diluant.

2. Esters d'acides 3-halogéno-3-cyano-2,2-diméthylpropane-1-carboxyliques de formule II:

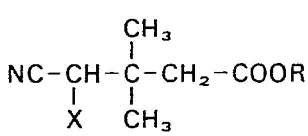
(II)

dans laquelle:
R représente un groupe alkyle éventuellement substitué, et
X représente un atome d'halogène.

3. Procédé de production d'esters d'acides 3-halogéno-3-cyano-2,2-diméthylpropane-1-carboxyliques de formule II:

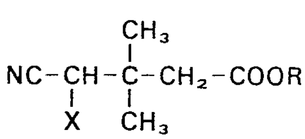
(II)

dans laquelle:
R représente un groupe alkyle éventuellement substitué, et
X représente un atome d'halogène,
caractérisé en ce que l'on fait réagir à des températures comprises entre −20 et +100°C un ester d'acide 3-carboxylique-3-cyano-2,2-diméthylpropane-1-carboxylique de formule III:

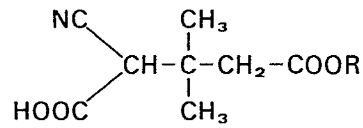
(III)

dans laquelle:
R a le sens précité,
avec un agent d'halogénation, éventuellement en présence d'un accepteur d'acide et éventuellement en utilisant un diluant, et l'on décarboxyle, par chauffage à des températures comprises entre 150 et 250°C, le produit d'halogénation, formé de manière intermédiaire et répondant à la formule IV:

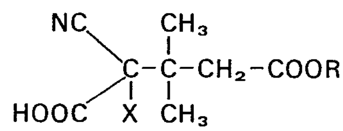
(IV)

dans laquelle:
R et X ont les sens précités.

4. Utilisation d'esters d'acides 3-halogéno-3-cyano-2,2-diméthylpropane-1-carboxyliques de formule II selon la revendication 2 pour produire des esters de l'acide 2-cyano-3,3-diméthylcyclopropane-1-carboxylique de formule I:

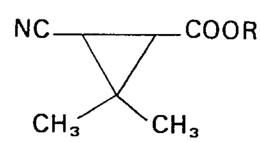
(I)

dans laquelle:
R représente un groupe alkyle éventuellement substitué,
caractérisé en ce qu'on fait réagir à des températures comprises entre 0 et 100°C un ester d'acide 3-halogéno-3-cyano-2,2-diméthylpropane-1-carboxylique de formule II:

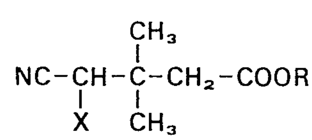
(II)

dans laquelle:
R a le sens précité, et
X représente un atome d'halogène,
avec une base, en opérant éventuellement en présence d'un diluant.